(19) **Europäisches Patentamt / European Patent Office / Office européen des brevets**

(11) **EP 4 382 095 A1**

(12) # EUROPEAN PATENT APPLICATION
published in accordance with Art. 153(4) EPC

(43) Date of publication:
**12.06.2024  Bulletin 2024/24**

(21) Application number: **22853550.6**

(22) Date of filing: **05.08.2022**

(51) International Patent Classification (IPC):
***A61K 9/16*** *(2006.01)*   ***A61K 9/00*** *(2006.01)*
***A61K 31/445*** *(2006.01)*   ***A61P 25/28*** *(2006.01)*

(52) Cooperative Patent Classification (CPC):
**A61K 9/00; A61K 9/16; A61K 31/445; A61P 25/28**

(86) International application number:
**PCT/KR2022/011672**

(87) International publication number:
**WO 2023/014175 (09.02.2023 Gazette 2023/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **05.08.2021  KR 20210103519**

(71) Applicant: **Whan In Pharmaceutical Co., Ltd.
Songpa-gu, Seoul 05855 (KR)**

(72) Inventors:
• **KIM, Byung-hyuck
Suwon-si Gyeonggi-do 16229 (KR)**

• **JUNG, Chan-eun
Suwon-si Gyeonggi-do 16229 (KR)**
• **KWON, Hyuk-il
Suwon-si Gyeonggi-do 16229 (KR)**
• **SHIN, Ho-chul
Suwon-si Gyeonggi-do 16229 (KR)**
• **HONG, Yong-soon
Suwon-si Gyeonggi-do 16229 (KR)**
• **KIM, Ju-hyun
Suwon-si Gyeonggi-do 16229 (KR)**

(74) Representative: **Louis Pöhlau Lohrentz
Patentanwälte
Postfach 30 55
90014 Nürnberg (DE)**

(54)  **SUSTAINED-RELEASE MICROSPHERES COMPRISING DONEPEZIL**

(57)  Disclosed is an injection including donepezil-containing microspheres.

EP 4 382 095 A1

**(Cont. next page)**

Donepezil SD rats PK drug concentration profile

[FIG. 1]

EP 4 382 095 A1

**Description**

TECHNICAL FIELD

The present invention relates to sustained-release microspheres containing donepezil and an injection including the same.

BACKGROUND ART

Donepezil is a drug developed to treat dementia (particularly, Alzheimer's dementia), and is currently sold as an oral tablet. The donepezil-containing oral tablet that is currently sold is directed to be taken once daily before bed.

However, since Alzheimer's patients have difficulty in taking the tablets on their own every day before bed, medication compliance may be low, and thus exhibiting a sustained pharmacological effect may be hindered.

Therefore, there is a need to develop a new dosage form to improve medication compliance when administering donepezil.

To solve this problem, research is being conducted to develop a sustained-release injection containing donepezil.

The sustained-release injection contains microspheres containing a drug included in a biodegradable polymer. When an injection containing such microspheres is injected into the body, the drug is slowly released from the injected microspheres, allowing the pharmacological effect to be exhibited continuously. Therefore, when a sustained-release injection is administered once, the drug can be released continuously for a long period of time, for example, at least one week or longer. If such a sustained-release injection is developed, the efficacy of a drug can continue even when the drug is administered by injection every one week, ten days, four weeks, or two months, thereby minimizing the number of times of invasion of the drug into the body and dramatically improving medication compliance.

To solve this problem, there is a need to develop a donepezil-containing sustained-release injection that minimizes fluctuation of blood drug concentration during the dosing cycle.

DETAILED DESCRIPTION OF THE INVENTION

TECHNICAL PROBLEM

An object of the present invention is to develop a donepezil-containing sustained-release injection that minimizes the fluctuation of blood drug concentration during administration, thereby allowing the pharmacological effects to be exhibited continuously while minimizing the occurrence of side effects.

TECHNICAL SOLUTION

The present invention may provide sustained-release microspheres releasing 40% to 60% of the total drug in the first half of a drug dosing cycle by preparing two dispersions including a polymer and having different drug loading amounts at a certain ratio and by preparing microspheres therefrom by, for example, a single emulsification method.

Specifically, the present invention relates to a sustained-release injection composition including two or more types of donepezil microspheres with different drug loading amounts, and the ratio of the AUC during the first half of the dosing cycle to the AUC during the dosing cycle (For example, it may be designed to be administered every week, every ten days, or every four weeks.) (i.e., $AUC_{0 \sim 1/2 \text{ dosing cycle}}$ / $AUC_{0 \sim \text{dosing cycle}}$) is 0.4 to 0.6. Here, the drug loading amount refers to the weight ratio of donepezil contained in the microspheres to the corresponding microspheres.

In the present invention, the dosing cycle may be designed to be, for example, seven days or more, preferably, it may have the range of seven days to two months. For example, it may be designed with the objective of administering the injection of the present invention every ten days or every four weeks.

In addition, in the present invention, the average drug loading of the donepezil microspheres is 35% or less, preferably 32% or less. To reduce the total dosage of the microspheres and lower the production cost during manufacturing, it may be advantageous to have a high drug loading amount, but when the drug loading amount is too high, the proportion of a polymer, which is a sustained-release agent, decreases, and accordingly, there is a problem that when the injection is administered, the blood drug concentration may not be maintained at an effective concentration during the dosing cycle, or the drug that is not encapsulated within the microspheres may exist as crystals, resulting in a high blood concentration immediately after administration.

In the present invention, the donepezil microspheres preferably include two types: those with a theoretical drug loading amount of 32% to 40% and those with a theoretical drug loading amount of 28 to 32%. That is, the injection of the present invention includes both donepezil-containing microspheres with a theoretical drug loading amount of 32% to 40% and donepezil-containing microspheres with a theoretical drug loading amount of 28% to 32% (Here, the present invention includes two or more types of donepezil microspheres of which drug loading amount is different from each other, and thus it is impossible that the theoretical drug loading amount of both types of microspheres is 32%.). Therefore, the injection of the present invention may include donepezil-containing microspheres with a theoretical drug loading amount of 28% or more to 32% or less and donepezil-containing microspheres with a theoretical drug loading amount of more than 32% to 40% or less, or alternatively, it may include donepezil-containing microspheres with a theoretical drug loading amount of 28% or more to less than 32% and donepezil-containing microspheres with a theoretical drug loading amount of 32% or more to 40% or less.

In the present specification, the term "theoretical drug loading amount" refers to the donepezil loading amount of donepezil in the microspheres when it is assumed that all of the input raw material donepezil and the input raw material polymer added when preparing donepezil-containing microspheres are prepared into microspheres and all of the input raw material donepezil is included within the microspheres, and it may be calculated from Mathematical Formula 1 below:

Theoretical drug loading amount (%) = [Amount of input donepezil when preparing microspheres/(Amount of input donepezil when preparing microspheres + Amount of input polymer when preparing microspheres)] × 100 (%)

In the present specification, the term "actual drug loading amount" refers to the amount of donepezil contained per 100 mg of actually prepared microspheres when donepezil-containing microspheres are prepared, and unless otherwise specified, it may be used interchangeably with the term "drug loading amount."

In the present invention, the weight ratio of microspheres with a theoretical drug loading amount of 32% to 40% to microspheres with a theoretical drug loading amount of 28% to 32% is preferably 1:0.8 to 5.

In addition, in the present invention, the donepezil microspheres include donepezil or a pharmaceutically acceptable salt, hydrate or solvate thereof, and a biodegradable polymer.

In the present invention, the donepezil microspheres preferably include an average of 25 to 32 mg of donepezil or a pharmaceutically acceptable salt thereof as donepezil 25 to 32 mg per 100 mg of microspheres (In other words, the actual drug loading amount in the present invention is preferably 25% to 32 %.).

In addition, the present invention relates to a method of preparing two or more types of donepezil microspheres with different drug loading amounts.

Donepezil-containing microspheres according to the present invention may be prepared by including: a step of preparing a first donepezil-containing dispersed phase having a first drug loading amount; a step of preparing a second donepezil-containing dispersed phase having a second drug loading amount; and a step of preparing microspheres by sequentially adding the first donepezil-containing dispersed phase and the second donepezil-containing dispersed phase to a continuous phase and stirring.

In addition, donepezil-containing microspheres according to the present invention may be prepared by including: a step of preparing a first donepezil-containing dispersed phase having a first drug loading amount; a step of preparing first microspheres by adding the first donepezil-containing dispersed phase to a continuous phase and stirring; a step of preparing a second donepezil-containing dispersed phase having a second drug loading amount; a step of preparing second microspheres by adding the second donepezil-containing dispersed phase to a separate continuous phase and stirring; and a step of mixing the first microspheres and the second microspheres.

In the method for preparing donepezil-containing microspheres of the present invention, the first drug loading amount and the second drug loading amount are different from each other.

In the present specification, the inclusion rate refers to the ratio of the actual drug loading amount to the theoretical drug loading amount, and is calculated according to Mathematical Formula 2 below:

[Mathematical Formula 2]

$$Inclusion\ rate = \frac{Actual\ drug\ loading\ amount}{Theoretical\ drug\ loading\ amount} \times 100\ (\%)$$

When actually preparing donepezil-containing microspheres, the inclusion rate has a value of approximately 90% to 100%.

Whether the drug loading amount refers to a target theoretical drug loading amount or an actual drug loading amount reflecting the inclusion rate, in the present invention, two or more types of microspheres showing different drug loading amounts may be included in an injection and administered, thereby exerting an effect of exhibiting a uniform AUC and a uniform drug concentration in blood during a dosing cycle.

ADVANTAGEOUS EFFECTS

The sustained-release microsphere injection according to the present invention may be prepared as two solutions having different drug loading amounts at a specific ratio by using polymers having different properties or simply by using a single polymer without an additional process so that a drug may be constantly released during a target period in the body, thereby suppressing side effects at a concentration higher than a therapeutic concentration and improving medication non-compliance.

DESCRIPTION OF THE DRAWINGS

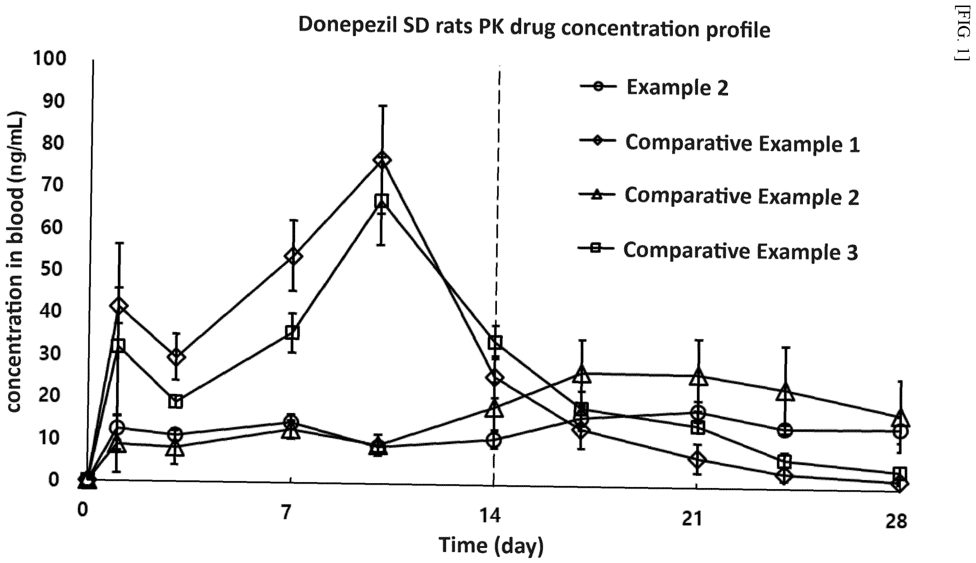

FIG. 1 shows the results of 28-day drug concentration profiles of Example 2 and Comparative Examples in an SD rat pharmacokinetic test of Experimental Example 3.
FIG. 2 is an SEM image to confirm the morphology of donepezil-containing microspheres prepared in Example 2.

MODE for INVENTION

Hereinafter, the present invention will be described in detail through examples to aid understanding. However, the following examples only illustrate the content of the present invention, and the scope of the present invention is not limited by the following examples. Examples of the present invention are provided to more completely explain the present invention to those with average knowledge in the art.

**Example 1:** Preparation of polymer microspheres having a theoretical drug loading amount of 36% and 30% in a weight ratio of 1:1 (total theoretical drug loading amount: 33%)

0.9 g of donepezil (manufacturer: Neuland Laboratories Co., Ltd., India) (36% of theoretical drug loading amount) and 1.6 g of poly D,L-lactide (Resomer R 203H; manufacturer: Evonik Co., Ltd., Germany), which is a biodegradable polymer, were added to 4.8 g of dichloromethane (manufacturer: Daejung Chemicals & Metals Co., Ltd., Korea) and stirred to completely dissolve, thereby preparing a first dispersed phase polymer solution.
Separately, 0.75 g of donepezil (manufacturer: Neuland Laboratories Co., Ltd., India) (30% of theoretical drug loading amount) and 1.75 g of D,L-lactide (Resomer R 203H; manufacturer: Evonik Co., Ltd., Germany), which is a biodegradable polymer, were added to 5.25 g of dichloromethane (manufacturer: Daejung Chemicals & Metals Co., Ltd., Korea) and stirred to completely dissolve, thereby preparing a second dispersed phase polymer solution.
In addition, an appropriate amount of polyvinyl alcohol was dissolved in water to prepare a 0.5% polyvinyl alcohol continuous phase.
The 0.5% polyvinyl alcohol continuous phase was placed in a doublejacketed beaker and maintained at a temperature below 10 °C using a constant temperature circulating water bath, and then the two dispersed phases prepared above (two types of donepezil-containing dispersed phase polymer solutions with different drug loading amounts) were each sequentially added to the continuous phase and stirred at a high speed to form an emulsion.
Then, to remove the organic solvent and obtain solidified microspheres, the organic solvent was volatilized at 47 °C for three hours and slowly cooled to 10 °C for one hour. The hardened microspheres were washed several times with water for injection, wet-filtered using a sieve, and freeze-dried to finally obtain microspheres containing donepezil.

**Example 2:** Preparation of polymer microspheres having a theoretical drug loading amount of 40% and 30% in a weight ratio of 1:4 (total theoretical drug loading amount: 32%)

1.6 g of donepezil (manufacturer: Neuland Laboratories Co., Ltd., India) (40% of theoretical drug loading amount) and 2.4 g of poly D,L-lactide (Resomer R 203H; manufacturer: Evonik Co., Ltd., Germany), which is a biodegradable polymer, were added to 7.2 g of dichloromethane (manufacturer: Daejung Chemicals & Metals Co., Ltd., Korea) and stirred to completely dissolve, thereby preparing a first dispersed phase polymer solution.
Separately, 4.8 g of donepezil (manufacturer: Neuland Laboratories Co., Ltd., India) (30% of theoretical drug loading amount) and 11.2 g of D,L-lactide (Resomer R 203H; manufacturer: Evonik Co., Ltd., Germany), which is a biodegradable polymer, were added to 33.6 g of dichloromethane (manufacturer: Daejung Chemicals & Metals Co., Ltd., Korea) and stirred to completely dissolve, thereby preparing a second dispersed phase polymer solution.
In addition, an appropriate amount of polyvinyl alcohol was dissolved in water to prepare a 0.5% polyvinyl alcohol continuous phase.
The 0.5% polyvinyl alcohol continuous phase was placed in a doublejacketed beaker and maintained at a temperature below 10 °C using a constant temperature circulating water bath, and then the two dispersed phases prepared above (two types of donepezil-containing dispersed phase polymer solutions with different drug loading amounts) were each sequentially added to the continuous phase and stirred at a high speed to form an emulsion.

Then, to remove the organic solvent and obtain solidified microspheres, the organic solvent was volatilized at 47 °C for three hours and slowly cooled to 10 °C for one hour. The hardened microspheres were washed several times with water for injection, wet-filtered using a sieve, and freeze-dried to finally obtain microspheres containing donepezil.

**Example 3:** Preparation of polymer microspheres having a theoretical drug loading amount of 38% and 28% in a weight ratio of 1:2 (total theoretical drug loading amount: 31.3%)

2.53 g of donepezil (manufacturer: Neuland Laboratories Co., Ltd., India) (38% of theoretical drug loading amount) and 4.13 g of poly D,L-lactide (Resomer R 203H; manufacturer: Evonik Co., Ltd., Germany), which is a biodegradable polymer, were added to 12.39 g of dichloromethane (manufacturer: Daejung Chemicals & Metals Co., Ltd., Korea) and stirred to completely dissolve, thereby preparing a first dispersed phase polymer solution.

Separately, 3.73 g of donepezil (manufacturer: Neuland Laboratories Co., Ltd., India) (30% of theoretical drug loading amount) and 9.6 g of D,L-lactide (Resomer R 203H; manufacturer: Evonik Co., Ltd., Germany), which is a biodegradable polymer, were added to 28.8 g of dichloromethane (manufacturer: Daejung Chemicals & Metals Co., Ltd., Korea) and stirred to completely dissolve, thereby preparing a second dispersed phase polymer solution.

In addition, an appropriate amount of polyvinyl alcohol was dissolved in water to prepare a 0.5% polyvinyl alcohol continuous phase.

The 0.5% polyvinyl alcohol continuous phase was placed in a doublejacketed beaker and maintained at a temperature below 10 °C using a constant temperature circulating water bath, and then the two dispersed phases prepared above (two types of donepezil-containing dispersed phase polymer solutions with different drug loading amounts) were each sequentially added to the continuous phase and stirred at a high speed to form an emulsion.

Then, to remove the organic solvent and obtain solidified microspheres, the organic solvent was volatilized at 47 °C for three hours and slowly cooled to 10 °C for one hour. The hardened microspheres were washed several times with water for injection, wet-filtered using a sieve, and freeze-dried to finally obtain microspheres containing donepezil.

**Example 4:** Preparation of polymer microspheres having a theoretical drug loading amount of 32% and 28% in a weight ratio of 1:1 (total theoretical drug loading amount: 30%)

3.2 g of donepezil (manufacturer: Neuland Laboratories Co., Ltd., India) (32% of theoretical drug loading amount) and 6.8 g of poly D,L-lactide (Resomer R 203H; manufacturer: Evonik Co., Ltd., Germany), which is a biodegradable polymer, were added to 20.4 g of dichloromethane (manufacturer: Daejung Chemicals & Metals Co., Ltd., Korea) and stirred to completely dissolve, thereby preparing a first dispersed phase polymer solution.

Separately, 2.8 g of donepezil (manufacturer: Neuland Laboratories Co., Ltd., India) (28% of theoretical drug loading amount) and 7.2 g of D,L-lactide (Resomer R 203H; manufacturer: Evonik Co., Ltd., Germany), which is a biodegradable polymer, were added to 21.6 g of dichloromethane (manufacturer: Daejung Chemicals & Metals Co., Ltd., Korea) and stirred to completely dissolve, thereby preparing a second dispersed phase polymer solution.

In addition, an appropriate amount of polyvinyl alcohol was dissolved in water to prepare a 0.5% polyvinyl alcohol continuous phase.

The 0.5% polyvinyl alcohol continuous phase was placed in a doublejacketed beaker and maintained at a temperature below 10 °C using a constant temperature circulating water bath, and then the two dispersed phases prepared above (two types of donepezil-containing dispersed phase polymer solutions with different drug loading amounts) were each sequentially added to the continuous phase and stirred at a high speed to form an emulsion.

Then, to remove the organic solvent and obtain solidified microspheres, the organic solvent was volatilized at 47 °C for three hours and slowly cooled to 10 °C for one hour. The hardened microspheres were washed several times with water for injection, wet-filtered using a sieve, and freeze-dried to finally obtain microspheres containing donepezil.

**Comparative Example 1:** Preparation of polymer microspheres having a theoretical drug loading amount of 40% in a single weight ratio

2 g of donepezil (manufacturer: Neuland Laboratories Co., Ltd., India) (40% of theoretical drug loading amount) and 3 g of poly D,L-lactide (Resomer R 203H; manufacturer: Evonik Co., Ltd., Germany), which is a biodegradable polymer, were added to 9 g of dichloromethane (manufacturer: Daejung Chemicals & Metals Co., Ltd., Korea) and stirred to completely dissolve, thereby preparing a dispersed phase polymer solution.

In addition, an appropriate amount of polyvinyl alcohol was dissolved in water to prepare a 0.5% polyvinyl alcohol continuous phase.

The 0.5% polyvinyl alcohol continuous phase was placed in a doublejacketed beaker and maintained at a temperature below 10 °C using a constant temperature circulating water bath, and then the dispersed phase prepared above (donepezil-containing dispersed phase polymer solution) was added to the continuous phase and stirred at a high speed to form an emulsion.

Then, to remove the organic solvent and obtain solidified microspheres, the organic solvent was volatilized at 47 °C for three hours and slowly cooled to 10 °C for one hour. The hardened microspheres were washed several times with water for injection, wet-filtered using a sieve, and freeze-dried to finally obtain microspheres containing donepezil.

**Comparative Example 2:** Preparation of polymer microspheres having a theoretical drug loading amount of 30% in a single weight ratio

6 g of donepezil (manufacturer: Neuland Laboratories Co., Ltd., India) (30% of theoretical drug loading amount) and 14 g of poly D,L-lactide (Resomer R 203H; manufacturer: Evonik Co., Ltd., Germany), which is a biodegradable polymer, were added to 42 g of dichloromethane (manufacturer: Daejung Chemicals & Metals Co., Ltd., Korea) and stirred to completely dissolve, thereby preparing a dispersed phase polymer solution.

In addition, an appropriate amount of polyvinyl alcohol was dissolved in water to prepare a 0.5% polyvinyl alcohol continuous phase.

The 0.5% polyvinyl alcohol continuous phase was placed in a doublejacketed beaker and maintained at a temperature below 10 °C using a constant temperature circulating water bath, and then the dispersed phase prepared above (donepezil-containing dispersed phase polymer solution) was added to the continuous phase and stirred at a high speed to form an emulsion.

Then, to remove the organic solvent and obtain solidified microspheres, the organic solvent was volatilized at 47 °C for three hours and slowly cooled to 10 °C for one hour. The hardened microspheres were washed several times with water for injection, wet-filtered using a sieve, and freeze-dried to finally obtain microspheres containing donepezil.

**Comparative Example 3:** Preparation of polymer microspheres having a theoretical drug loading amount of 40% and 30% in a weight ratio of 1:1 (total theoretical drug loading amount: 35%)

1 g of donepezil (manufacturer: Neuland Laboratories Co., Ltd., India) (40% of theoretical drug loading amount) and 1.5 g of poly D,L-lactide (Resomer R 203H; manufacturer: Evonik Co., Ltd., Germany), which is a biodegradable polymer, were added to 4.5 g of dichloromethane (manufacturer: Daejung Chemicals & Metals Co., Ltd., Korea) and stirred to completely dissolve, thereby preparing a first dispersed phase polymer solution.

Separately, 0.75 g of donepezil (manufacturer: Neuland Laboratories Co., Ltd., India) (30% of theoretical drug loading amount) and 1.75 g of D,L-lactide (Resomer R 203H; manufacturer: Evonik Co., Ltd., Germany), which is a biodegradable polymer, were added to 5.25 g of dichloromethane (manufacturer: Daejung Chemicals & Metals Co., Ltd., Korea) and stirred to completely dissolve, thereby preparing a second dispersed phase polymer solution.

In addition, an appropriate amount of polyvinyl alcohol was dissolved in water to prepare a 0.5% polyvinyl alcohol continuous phase.

The 0.5% polyvinyl alcohol continuous phase was placed in a doublejacketed beaker and maintained at a temperature below 10 °C using a constant temperature circulating water bath, and then the two dispersed phases prepared above (two types of donepezil-containing dispersed phase polymer solutions with different drug loading amounts) were each sequentially added to the continuous phase and stirred at a high speed to form an emulsion.

Then, to remove the organic solvent and obtain solidified microspheres, the organic solvent was volatilized at 47 °C for three hours and slowly cooled to 10 °C for one hour. The hardened microspheres were washed several times with water for injection, wet-filtered using a sieve, and freeze-dried to finally obtain microspheres containing donepezil.

**Experimental Example 1:** Observation of microsphere morphology using SEM

Approximately 20 mg of microspheres were fixed to an aluminum stub using a carbon tape, coated with platinum for three minutes under a vacuum degree of 0.1 Torr and a high voltage (10 kV), and then mounted on a scanning electron microscopy (SEM, equipment name: SEC-SNE 4500M Plus A, Korea), and the surface morphology of the microspheres was observed using an image analysis software program (mini-SEM).

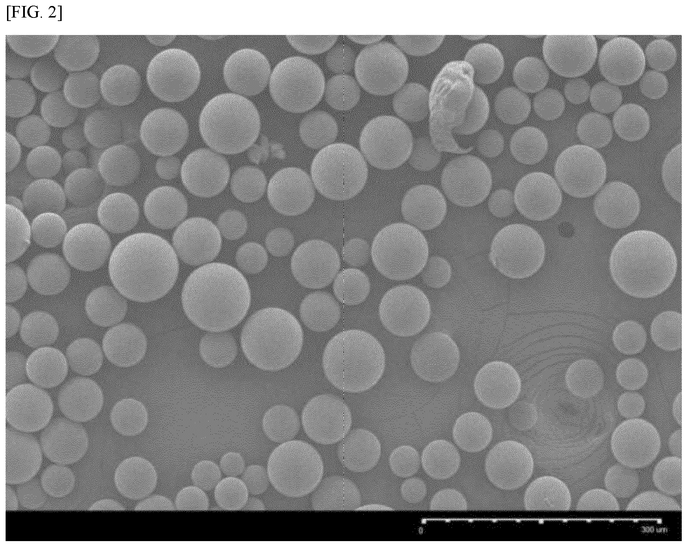

FIG. 2 is a SEM image of the microspheres prepared in example 1.

No unusual features were found in the microspheres prepared in Examples 1 to 4 and Comparative Examples 1 to 3.

**Experimental Example 2:** Measurement of inclusion rate and content of donepezil in microspheres

For each of the microspheres prepared in Examples 1 to 4 and Comparative Examples 1 to 3, about 100 mg was taken, completely dissolved in acetonitrile, and then diluted with a mobile phase. 20 µL of the diluted solution was injected into the high-performance liquid chromatography (HPLC), and the absorbance was measured at a detection wavelength of 318 nm.

<Measurement conditions>

Column: Luna phenyl-hetyl, C18 5 µm, 4.6 × 250 mm

Mobile phase: a 3:1 mixed solution of a solution of tetrahydrofuran and triethylamine (solution A) and a solution of methanol and tetrahydrofuran (solution B) (pH 2.0)

Each of the actual drug loading amount measured (amount of donepezil present per 100 mg of microspheres) and the drug inclusion rate are shown in Table 1 below.

[Table 1]

|  | Ratio of theoretical drug loading amounts | Average theoretical drug loading amount (%) | Drug inclusion rate (%) | Actual drug loading amount (%) |
|---|---|---|---|---|
| Example 1 | 36%:30% = 1:1 | 33.0 | 92.9 | 30.65 |
| Example 2 | 40%:30% = 1:4 | 32.0 | 94.2 | 30.16 |
| Example 3 | 38%:28% = 1:2 | 31.3 | 94.8 | 29.71 |
| Example 4 | 32%:28% = 1:1 | 30.0 | 95.6 | 28.68 |
| Comparative Example 1 | 40% single | 40.0 | 94.4 | 37.74 |
| Comparative Example 2 | 30% single | 30.0 | 89.6 | 26.88 |
| Comparative Example 3 | 40%:30% = 1:1 | 35.0 | 91.6 | 32.07 |

As described above, the inclusion rate is calculated according to the following mathematical formula:

[Mathematical Formula 2]

$$Inclusion\ rate = \frac{Actual\ drug\ loading\ amount}{Theoretical\ drug\ loading\ amount} \times 100\ (\%)$$

In the above table, "ratio of theoretical drug loading amounts" represents the ratio of the total weight of microspheres with a relatively high theoretical drug loading amount to the total weight of microspheres with a relatively low theoretical drug loading amount.
The drug encapsulation rates of Examples 1 to 4 and Comparative Examples 1 to 3 were all found to be about 90% or more.

**Experimental Example 3:** Pharmacokinetic test of microsphere injection using Sprague-Daweley (SD) rats

The present experiment is intended to confirm the effect of reducing the fluctuation of the blood drug concentration depending on the content ratio of microspheres with different drug loading amounts.

The microspheres prepared in Examples 1 to 4 and Comparative Examples 1 to 3 were administered by subcutaneous injection to the dorsal part of SD rats, and the concentration of donepezil in the blood was measured.

Specifically, microspheres corresponding to 25.2 mg of donepezil per SD rat were administered by subcutaneous injection to the dorsal part of SD rats (n=6).

On days 1, 3, 7, 10, 14, 17, 21, 24, 28, and 35 days after the injection, 0.3 mL of blood was collected from the rat's jugular vein, kept in ice-cold conditions, and centrifuged to separate 100 $\mu$L of plasma. The concentration of donepezil in the separated plasma was analyzed using LC/MS/MS. The results of the pharmacokinetic test on rats are shown in Table 2.

[Table 2]

| | $AUC_{0-336h}$ (hr*ng/mL) | $AUC_{0-672h}$ (hr*ng/mL) | $AUC_{0-336h}/AUC_{0-672h}$ ratio |
|---|---|---|---|
| Example 1 | 5120.4 | 9288.6 | 0.55 |
| Example 2 | 6055.7 | 11639.2 | 0.52 |
| Example 3 | 5871.3 | 9997.4 | 0.59 |
| Example 4 | 4056.0 | 9209.1 | 0.44 |
| Comparative Example 1 | 17433.3 | 20335.6 | 0.86 |
| Comparative Example 2 | 3705.5 | 11651.9 | 0.32 |
| Comparative Example 3 | 13297.3 | 17602.1 | 0.76 |

As shown in Table 2, in the cases of Comparative Examples 1 and 2 containing only microspheres with a drug loading amount of 30% or 40%, the ratio of the AUC up to 336 hr (14 days), the first half of the dosing cycle, to the AUC up to 672 hr (28 days), the target dosing cycle, was 0.86 and 0.32, respectively.

The area under curve (AUC) is a parameter related to the amount of drug absorbed from the digestive tract or from tissues upon injection and reaching the body's circulating bloodstream. In Comparative Example 1, when administered by injection, an excessively large amount of the drug reaches the bloodstream during the first half of the dosing cycle, making it difficult to reach an effective blood concentration after the first half of the dosing cycle. In Comparative Example 2, when administered by injection, a relatively small amount of the drug reaches the bloodstream during the first half of the dosing cycle, so an effective blood concentration may not be reached during the first half of the dosing cycle.

On the other hand, in the case of Examples 1 to 4 including two types of microspheres with different drug loading amounts, the ratio of the AUC up to 336 hr (14 days), the first half of the dosing cycle, to the AUC up to 672 hr (28 days), the target dosing cycle, had the range of 0.4 to 0.6. In other words, a uniform AUC is exhibited during the target dosing cycle, and drug release is exhibited uniformly. Therefore, when the microspheres of Examples 1 to 4 are administered by injection, an effective blood concentration can be continuously reached throughout the dosing cycle, and a phenomenon in which an abnormally low blood drug concentration or an abnormally high blood drug concentration is exhibited in the early stage of administration so that no therapeutic effect is exhibited or a biased therapeutic effect is exhibited may be prevented. Furthermore, medication compliance can may be improved.

However, in the cases where two types of microspheres with different drug loading amounts were included, and the theoretical drug loading amount of the microspheres with a relatively high theoretical drug loading amount was relatively high such as 40%, it was preferable that the total weight of the microspheres with a relatively low theoretical drug loading was larger. That is, in the case of Example 2 where the total weight ratio of the microspheres with a theoretical drug loading amount of 40% to the microspheres with a theoretical drug loading amount of 30% was 1:4, the average actual drug loading amount of the microspheres was 30%, which was relatively small, and the AUC ratio was 0.52, which was a preferable result. However, in the case of Comparative Example 3 where the total weight ratio of the microspheres with a theoretical drug loading amount of 40% to the microspheres with a theoretical drug loading amount of 30% was 1: 1, the average actual drug loading amount of the microspheres was 32.07%, which was relatively large, and the AUC ratio was 0.76, which was a slightly insufficient result.

Therefore, in could be seen that in the cases where two types of microspheres with different drug loading amounts were included, and the theoretical drug loading amount of the microspheres with a relatively high theoretical drug loading amount was 40%, a slightly small effect was exhibited when the average actual drug loading amount of the microspheres exceeded 32%, and a preferable effect was exhibited when the average actual drug loading amount of the microspheres was 32% or less. Therefore, in the present invention, in the cases where the amount of the microspheres with a relatively high drug loading amount is relatively high among two types of microspheres with different drug loading amounts, it is preferable that the average actual drug loading amount of the microspheres is 32% or less.

**Experimental Example 4:** Pharmacokinetic test of microspheres using beagles

This experiment is intended to confirm whether the results of the AUC ratio in the pharmacokinetics (PK) test for SD rats in Experiment Example 3 are similarly exhibited in beagle dogs, which are another experimental animal species.

Similar to Experimental Example 3, the microspheres prepared in Examples 1 to 4 and Comparative Examples 1 to 3 were each administered by intramuscular injection into the thigh region of a beagle dog, and the concentration of donepezil in the blood was measured.

Specifically, microspheres corresponding to 84.0 mg of donepezil per beagle dog were injected intramuscularly into the thigh region of each beagle dog (n=3).

On days 1, 3, 7, 10, 14, 17, 21, 24, 28, and 35 days after the injection, 0.5 mL of blood was collected from the cephalic vein of a beagle dog, kept in ice-cold conditions, and centrifuged to separate 100 $\mu$L of plasma. The concentration of donepezil in the separated plasma was analyzed using LC/MS/MS. The results of the pharmacokinetic test on beagle dogs are shown in Table 3.

[Table 3]

|  | $AUC_{0-336h}$ (hr*ng/mL) | $AUC_{0-672h}$ (hr*ng/mL) | $AUC_{0-336h}/AUC_{0-672h}$ ratio |
|---|---|---|---|
| Example 2 | 1167.9 | 2038.9 | 0.57 |
| Example 3 | 1205.6 | 2168.0 | 0.56 |
| Example 4 | 795.8 | 1629.7 | 0.49 |

As shown in Table 3, after administering Examples 2 to 4 to the beagle dogs, the $AUC_{0-336h}/AUC_{0-672h}$ ratio was within the range of 0.4 to 0.6, which is indicative of uniform drug release as in the case of rat administration.

Therefore, it was confirmed that the microsphere-containing injection composition according to the present invention can exhibit a constant therapeutic effect during the target dosing cycle by showing a constant AUC during the dosing cycle, and that this effect appears similarly even when administered to other species.

INDUSTRIAL APPLICABILITY

The injection composition according to the present invention can be effectively used as a therapeutic agent for dementia.

**Claims**

1. A sustained-release injection composition comprising two or more types of donepezil microspheres with different drug loading amounts, wherein the ratio of AUC during a first half of a dosing cycle to AUC during the dosing cycle is 0.4 to 0.6
(Here, the drug loading amount refers to a weight ratio of donepezil contained in each type of microspheres to corresponding type of microspheres.)

2. The injection composition according to claim 1, wherein the dosing cycle is one week to two months.

3. The injection composition according to claim 1, wherein the dosing cycle is 10 days to 28 days.

4. The injection composition according to claim 1, wherein the donepezil microspheres include two types: those with a theoretical drug loading amount of 32% to 40% and those with a theoretical drug loading amount of 28 to 32%.

5. The injection composition according to claim 1, wherein an average drug loading amount is 32% or less.

6. The injection composition according to claim 4, wherein a weight ratio of microspheres with a theoretical drug loading amount of 32% to 40% to microspheres with a theoretical drug loading amount of 28% to 32% is 1:0.8 to 5.

7. The injection composition according to any one of claims 1 to 6, wherein the donepezil microspheres include donepezil or a pharmaceutically acceptable salt, hydrate or solvate thereof, and a biodegradable polymer.

8. The injection composition according to claim 5, wherein the donepezil microspheres include an average of 25 to 32 mg of donepezil or a pharmaceutically acceptable salt thereof as donepezil 25 to 32 mg per 100 mg of microspheres.

9. A method of preparing donepezil microspheres, the method comprising:

a step of preparing a first donepezil-containing dispersed phase having a first drug loading amount;
a step of preparing a second donepezil-containing dispersed phase having a second drug loading amount; and
a step of preparing microspheres by sequentially adding the first donepezil-containing dispersed phase and the second donepezil-containing dispersed phase to a continuous phase and stirring,
wherein the first drug loading amount and the second drug loading amount are different from each other to prepare donepezil microspheres with different drug loading amounts.

10. A method of preparing donepezil microspheres, the method comprising:

a step of preparing a first donepezil-containing dispersed phase having a first drug loading amount;
a step of preparing first microspheres by adding the first donepezil-containing dispersed phase to a continuous phase and stirring;
a step of preparing a second donepezil-containing dispersed phase having a second drug loading amount;
a step of preparing second microspheres by adding the second donepezil-containing dispersed phase to a separate continuous phase and stirring; and
a step of mixing the first microspheres and the second microspheres,
wherein the first drug loading amount and the second drug loading amount are different from each other to prepare donepezil microspheres with different drug loading amounts.

[FIG. 1]

Donepezil SD rats PK drug concentration profile

Example 2
Comparative Example 1
Comparative Example 2
Comparative Example 3

[FIG. 2]

## INTERNATIONAL SEARCH REPORT

International application No.

**PCT/KR2022/011672**

### A.    CLASSIFICATION OF SUBJECT MATTER

**A61K 9/16**(2006.01)i; **A61K 9/00**(2006.01)i; **A61K 31/445**(2006.01)i; **A61P 25/28**(2006.01)i

According to International Patent Classification (IPC) or to both national classification and IPC

### B.    FIELDS SEARCHED

Minimum documentation searched (classification system followed by classification symbols)

A61K 9/16(2006.01); A61K 31/445(2006.01); A61K 39/00(2006.01); A61K 47/68(2017.01); A61K 9/00(2006.01); C07K 16/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 미립구(microsphere), 약물(drug), 도네페질(donepezil)

### C.    DOCUMENTS CONSIDERED TO BE RELEVANT

| Category* | Citation of document, with indication, where appropriate, of the relevant passages | Relevant to claim No. |
|---|---|---|
| X | KR 10-2019-0064526 A (G2GBIO, INC.) 10 June 2019 (2019-06-10)<br>See claims 1-8; and paragraphs [0093]-[0099] and [0135]-[0137]. | 1-10 |
| A | KR 10-2018-0088445 A (ABBVIE STEMCENTRX LLC) 03 August 2018 (2018-08-03)<br>See claims 1-28; and paragraph [0442]. | 1-10 |
| A | KR 10-2212717 B1 (WHAN IN PHARMACEUTICAL CO., LTD.) 08 February 2021 (2021-02-08)<br>See claim 1; and paragraphs [0034] and [0079]. | 1-10 |
| A | KR 10-2019-0078017 A (DONG KOOK PHARM. CO., LTD.) 04 July 2019 (2019-07-04)<br>See entire document. | 1-10 |
| A | KR 10-2016-0070191 A (STEMCENTRX, INC.) 17 June 2016 (2016-06-17)<br>See entire document. | 1-10 |

☐ Further documents are listed in the continuation of Box C.        ☑ See patent family annex.

| | |
|---|---|
| *    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **17 November 2022** | **18 November 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.

**PCT/KR2022/011672**

| Patent document cited in search report | | | Publication date (day/month/year) | Patent family member(s) | | | Publication date (day/month/year) |
|---|---|---|---|---|---|---|---|
| KR | 10-2019-0064526 | A | 10 June 2019 | CN | 110582270 | A | 17 December 2019 |
| | | | | EP | 3590504 | A1 | 08 January 2020 |
| | | | | JP | 2020-524171 | A | 13 August 2020 |
| | | | | JP | 6905767 | B2 | 21 July 2021 |
| | | | | KR | 10-2047982 | B1 | 22 November 2019 |
| | | | | US | 2020-0101054 | A1 | 02 April 2020 |
| | | | | WO | 2019-108029 | A1 | 06 June 2019 |
| | | | | ZA | 201808115 | B | 28 August 2019 |
| KR | 10-2018-0088445 | A | 03 August 2018 | CA | 3006738 | A1 | 08 June 2017 |
| | | | | CL | 2018001481 | A1 | 24 August 2018 |
| | | | | CL | 2019000189 | A1 | 07 June 2019 |
| | | | | CN | 108473588 | A | 31 August 2018 |
| | | | | EP | 3383917 | A1 | 10 October 2018 |
| | | | | JP | 2019-500335 | A | 10 January 2019 |
| | | | | US | 2019-0083645 | A1 | 21 March 2019 |
| | | | | WO | 2017-096163 | A1 | 08 June 2017 |
| KR | 10-2212717 | B1 | 08 February 2021 | EP | 4056170 | A1 | 14 September 2022 |
| | | | | WO | 2021-091333 | A1 | 14 May 2021 |
| KR | 10-2019-0078017 | A | 04 July 2019 | None | | | |
| KR | 10-2016-0070191 | A | 17 June 2016 | CA | 2928671 | A1 | 14 May 2015 |
| | | | | CA | 2966618 | A1 | 12 May 2016 |
| | | | | CL | 2016001102 | A1 | 10 February 2017 |
| | | | | CL | 2017001118 | A1 | 05 January 2018 |
| | | | | CN | 105813650 | A | 27 July 2016 |
| | | | | CN | 107207580 | A | 26 September 2017 |
| | | | | CO | 2017005538 | A2 | 10 October 2017 |
| | | | | EP | 3065776 | A2 | 14 September 2016 |
| | | | | EP | 3215523 | A1 | 13 September 2017 |
| | | | | JP | 2016-536020 | A | 24 November 2016 |
| | | | | JP | 2017-535283 | A | 30 November 2017 |
| | | | | JP | 6515111 | B2 | 22 May 2019 |
| | | | | KR | 10-2017-0085531 | A | 24 July 2017 |
| | | | | MA | 40921 | A | 12 September 2017 |
| | | | | MX | 2016005763 | A | 19 August 2016 |
| | | | | MX | 2017005797 | A | 23 October 2017 |
| | | | | PE | 08702016 | A1 | 09 September 2016 |
| | | | | PE | 10602017 | A1 | 21 July 2017 |
| | | | | PE | 20160870 | A1 | 09 September 2016 |
| | | | | PE | 20171060 | A1 | 21 July 2017 |
| | | | | PH | 12016500781 | A1 | 13 June 2016 |
| | | | | PH | 12017500825 | A1 | 18 October 2017 |
| | | | | RU | 2016122041 | A | 11 December 2017 |
| | | | | RU | 2016122041 | A3 | 27 March 2018 |
| | | | | SG | 11201603397 | A | 30 May 2016 |
| | | | | SG | 11201703669 | A | 29 June 2017 |
| | | | | TW | 201625677 | A | 16 July 2016 |
| | | | | US | 10053511 | B2 | 21 August 2018 |
| | | | | US | 2016-0289332 | A1 | 06 October 2016 |
| | | | | US | 2017-0334991 | A1 | 23 November 2017 |
| | | | | US | 2019-0077876 | A1 | 14 March 2019 |

Form PCT/ISA/210 (patent family annex) (July 2022)

**INTERNATIONAL SEARCH REPORT**
Information on patent family members

International application No.

**PCT/KR2022/011672**

| Patent document cited in search report | Publication date (day/month/year) | Patent family member(s) | | Publication date (day/month/year) |
|---|---|---|---|---|
| | | WO | 2015-069794 A2 | 14 May 2015 |
| | | WO | 2015-069794 A3 | 18 June 2015 |
| | | WO | 2015-069794 A9 | 14 May 2015 |
| | | WO | 2016-073649 A1 | 12 May 2016 |

Form PCT/ISA/210 (patent family annex) (July 2022)